# EUROPÄISCHE PATENTANMELDUNG

(11) **EP 0 765 633 A1**
(43) Veröffentlichungstag der Anmeldung: **02.04.1997**
(21) Anmeldenummer: 96115333.5
(22) Anmeldetag: 25.09.1996
(51) Int. Cl.: A61B 5/103

(54) **Vorrichtung zur Erzeugung von Temperaturwechseln**

(30) Priorität: 26.09.1995 DE 19535640
(71) Anmelder: FORSCHUNGSZENTRUM JÜLICH GMBH, 52425 Jülich (DE)
(72) Erfinder: Butzek, Michael, 52078 Aachen (DE); Halling, Horst, Prof., 52459 Pier (DE); Meixner, Christoph, Dr., 52428 Jülich (DE); Renftle, Walter, 52353 Düren (DE); Schmitt, Gisela, Dr., 52064 Aachen (DE)

(57) **Zusammenfassung**

Eine Vorrichtung zur Erzeugung von Temperaturwechseln weist ein Kontaktmittel (1) auf, das an der Probe (13) befestigt wird. Zumindest zwei verschiedene Gase oder Flüssigkeiten werden in Vorratsbehältem (2,3) temperiert und einer Umschalteinrichtung zeitgleich zugeführt. Die Umschalteinrichtung (8) hat verschiedene Ausgänge, versehen mit Schlauchverbindungen (9,10,11). Die beiden wodurch die Flüssigkeiten getrennt und gleichzeitig abfliessen können. Eine Schlauchverbindungen (9) ist an das Kontaktmittel (1) angeschlossen. Wird umgeschaltet, so fließt eine anders temperierte Flüssigkeit durch das Kontaktmittel hindurch. So ist es möglich, hochfrequente Temperaturwechsel zu erzeugen.

Die Vorrichtung ist insbesondere in der Schmerzerforschung sowie in der Schmerzdiagnostik einsetzbar.

## Beschreibung

Die Erfindung bezieht sich auf eine Vorrichtung zur Erzeugung von Temperaturwechseln an einer Probe mittels eines Kontaktmittels.

Bekannt sind beispielsweise um einen Metallblock gewickelte Heizspiralen (Kontaktmittel). Der Metallblock ist mit dem zu erwärmenden Körper (Probe) thermisch leitend verbunden. Soll der Körper erhitzt werden so wird Strom durch die Heizspiralen geschickt, die sich infolgedessen erhitzen. Über den Metallblock wird die Wärme auf den Körper übertragen und eine Temperaturveränderung (Temperaturwechsel) des Körpers bewirkt.

Eine derartige Vorrichtung eignet sich aufgrund der Trägheit nicht für schnelle Temperaturwechsel, insbesondere nicht für ständiges schnelles Hin- und Herwechseln zwischen verschiedenen Temperaturen.

Es ist beispielsweise auf dem Gebiet der Diagnostik und Therapie chronischer Schmerzsyndrome beim Menschen von Interesse, schnell zwischen zwei Temperaturen hin- und herwechseln zu können. Auf diese Weise könnten thermische Schmerzstimulationen durchgeführt werden (Handwerker, H.O., Kobal, G., Psychophysiology of experimentally induced pain, Physiological Rev. Vol. 73, S. 639,1993). Bis heute steht jedoch keine geeignete Vorrichtung zur Verfügung.

Das subjektive Schmerzerleben wird als Ereignis betrachtet, das sich als komplexe Hirnfunktion verstehen laßt (Loeser, J.D., What is chronic pain, Theoretical Medicine Vol. 12, S. 213, 1991). Daher werden Techniken zur Untersuchung der Hirnfunktion, z.B. die Magnetoenzephalographie (MEG), die funktionelle Kernspintomographie (fMRI) und die Positronen-Emissions-Tomographie (PET) - kurz als bildgebende Verfahren' oder ,imaging'- Techniken bezeichnet - nun auch in Fragestellungen der Schmerzforschung eingesetzt. Untersuchungen mittels MEG und fMRI sind von besonderem Interesse, da aufgrund der Möglichkeiten der zeitlichen und räumlichen Auflösung beider Verfahren (msec und mm) Hirnaktivitäten sozusagen in real-time'' abgebildet werden können.

Es ist erforderlich, die Randbedingungen der Verfahren aufeinander abzustimmen. Bei Untersuchungen mittels MEG und FMRI sind beispielsweise alle Gerätekomponenten, die magnetische Felder erzeugen oder von magnetischen Feldern gestört werden können, aus dem Untersuchungsbereich fernzuhalten.

Die momentan zur Verfügung stehenden experimentellen Schmerzstimulationsverfahren genügen den oben genannten Bedingungen bei weitem nicht. Zur thermischen Schmerzstimulation wird z.B. weltweit die sog. Marstock'-Thermode (Fruhstorfer, H., Lindblom, U., Schmidt, W.G., Method for quantitative estimation of thermal thresholds in patients, J. Neurol. Neurosurg. PsychiatryVol. 39, S. 1071, 1976; Lautenbacher, S., Galfe, G., Hölzl, R., Strian, F., Threshold tracking for assessment of long-term adaptation and sensitization in pain perception, Percept. Mot. Skills Vol. 69, S. 579, 1989b; Claus, D.., Hilz, M.J., Hummer, 1., Neundörfer, B., Methods of measurement of thermal Thresholds, Acta Neuroi.Scand. Vol. 76, S. 288, 1987; Claus, D.., Hilz, M.J., Neundörfer, B., Thermal discrimination Thresholds: a comparison of different methods, Acta Neurol.Scand. Vol. 81, S. 533, 1990), ein auf Basis eines stromgesteuerten Peltier-Elementes arbeitendes Verfahren, eingesetzt. Nach diesem Prinzip arbeitende Geräte sind jedoch weder von der zeitlichen Stimulationscharakteristik (maximaler Temperaturgradient 2 °C/sec), noch von den Materialeigenschaften und dem verwendeten Energieträger her interessant oder geeignet für die Untersuchung schmerzspezifischer Hirnaktivität in magnetisch sensibler Umgebung.

Erforderlich wäre hierfür eine Vorrichtung, mit der folgende Anforderungen erfüllt werden können:
- Die Geschwindigkeit des Gerätes soll eine Stimulation bis zu 10 Hz bei einer Amplitude von mindestens 5 °C gestatten. Als Signalform soll dabei ein möglichst steilflankiges Trapez erreicht werden. Wird ein Flankenanteil des Trapezsignals von 10% angenommen, so ergibt sich ein zu erreichender Temperaturgradient von 500 °C/sec oder dem 250-fachen Wert des zur Zeit verwendeten Gerätes auf der Basis eines Peltier-Element.
   - Die Verfahren MEG und FMRI werden in hochgradig magnetisch abgeschirmten Räumen durchgeführt. Die innerhalb dieser Kammern eingesetzten Teile des zu entwickelnden Gerätes dürfen weder Magnetfelder erzeugen (MEG) noch Verändern (MEG) oder sensibel auf starke und schnell wechselnde Magnetfelder im Teslabereich (FMRI) reagieren. Der Einsatz von elektrischem Strom und die Verwendung jeglicher Art von elektrisch leitfahigem Material, insbesondere Metall ist somit nicht möglich.

Aufgrund der geforderten Geschwindigkeit des Gerätes müssen relativ große (thermische) Energiemengen bewegt werden. Als magnetisch unbedenkliche Energieträger bieten sich (Laser-) licht und Fluide an. Der Laser hat den Nachteil, daß sich mit ihm Energie nur zu- nicht aber abführen läßt. Wird die Energie mittels eines zusätzlichen Fluidstromes abgeführt, so sind Laserleistungen von ca. 5 kW notwendig. Wird der Laser nur zur punktuellen Erhitzung eines einzelnen Nervs eingesetzt, so vermindert sich zwar das Kühlproblem, die erzeugte Stimulation entspricht aber keinem relevanten klinischen Schmerzmodell und liefert somit keine brauchbaren Meßergebnisse.

Aufgabe der Erfindung ist die Schaffung einer Vorrichtung zur schnellen Erzeugung von Temperaturwechseln, die bei geeigneter Ausgestaltung eine thermische Schmerzsimulation gemäß vorgenanntem Anforderungsprofil ermöglicht.

Gelöst wird die Aufgabe durch eine Vorrichtung mit den Merkmalen des Anspruchs 1. Vorteilhafte Ausgestaltungen zur Lösung der Aufgabe ergeben sich aus den Unteransprüchen.

Die Vorrichtung funktioniert wie folgt: Verschiedene Flüssigkeiten oder Gase werden (durch Temperierungsmittel) auf unterschiedliche Temperaturen gebracht und strömen in die Einrichtung hinein (mittels der Zuleitungen) und aus ihr wieder heraus (mittels der Ableitungen). Einer der temperierten Gas- oder Flüssigkeitsströme durchströmt das Kontaktmittel, die übrigen fließen irgendwo anders hin.

Soll die Temperatur an der Probe gewechselt werden, so wird innerhalb der Einrichtung derart umgeschaltet (mittels Umschaltmittel), daß ein anders temperierter Gas- oder Flüssigkeitsstrom den Kontaktkopf durchströmt. Die Umschaltmittel bewirken also, daß sich die Verbindung zwischen Zu- und Ableitungen verändern. Der Kontaktkopf und damit die Probe wird so auf eine andere Temperatur gebracht. Durch erneutes Umschalten können auf schnelle Art und Weise neue Temperaturen an der Probe erreicht werden. Hochfrequentes Hin- und Herschalten der Umschaltmittel bewirken hochfrequente Temperaturwechsel, entsprechend den Erfordernissen bei Hirnforschung am Modellschmerz. Auch können sämtliche (magnet-)feldbeeinflußende Teile vorteilhaft problemlos ausgelagert werden.

Als Kontaktmittel ist insbesondere ein zu einer Seite hin offener Behälter vorgesehen, dessen offene Seite an der Probe befestigt wird. Durch den so hergestellten unmittelbaren Kontakt zwischen Probe und den, jeweiligen temperierten Flüssigkeits- oder Gasstrom wird die gewünschte Wärmeübertragung maximiert. Dieses Kontaktmittel eignet sich für jede Vorrichtung, bei der mittels Flüssigkeiten oder Gase Wärme oder Kälte auf eine Probe übertragen werden soll. Selbstverständlich ist sie auch geeignet, um Proben lokal mit Flüssigkeiten zu benetzen.

Als Befestigungsmittel eignen sich vorteilhaft Mittel zum Herauspumpen von Gasen oder Flüssigkeiten, die an den Behälter angeschlossen sind. Die offene Seite des Behälters wird auf die Probe aufgesetzt. Durch die Pumpmittel wird ein Unterdruck im Behälter erzeugt. Durch den Unterdruck wird der Behälter an die Probe angesaugt. Diese Art der Befestigung hat den Vorteil, schnell und einfach die Befestigung sowie das anschließende Loslösen des Behälters zu ermöglichen.

Zur Vermeidung von Leckagen zwischen Behälter und Probe können zweckmäßig Dicht- oder Klebemittel an den Übergangsstellen eingesetzt werden. Ferner kann der Behälter mit weiteren Ansaugmitteln versehen werden, die den Behälter mittels Unterdruck an der Probe fixieren.

Der gewünschte Umschaltvorgang wird insbesondere durch Ventile zum Umschalten zwischen Fluiden bewerkstelligt, die Einlaß- und Auslaßöffnungen entlang zu einer im Ventil befindlichen Welle sowie derart an der Welle angebrachten Leitblechen, daß in das Ventil hineinströmende Fluide durch Drehen der Welle zu einer anderen Auslaßöffnung als vor der Drehung umgelenkt werden können, aufweisen. Gegenüber konventionellen Umschaltventilen zwischen zwei oder mehreren Fluiden weisen diese Ventile folgende Vorzüge auf:
- Das Umschalten der Fluidströme erfolgt vibrationsfrei.
- Speziell bei inkompressiblen Fluiden dürfen die Zuflüsse während des Schaltvorganges nicht blockiert werden. Würde das Fluid in einer langen Zuflußleitung durch den Umschaltvorgang in seiner Geschwindigkeit gebremst, so wäre eine hohe Frequenz des Schaltvorgangs aufgrund der Massenträgheit der Fluidsäule vor dem Zulauf unmöglich. Dieses Problem wird durch das vorgenannte Ventil gelöst.
- Ein steilflankiges Umschalten ist erreichbar, d. h. es findet kein allmählicher Wechsel von einem Fluid zu einem anderen statt. Es wird also schnellstmöglich von einer Flüssigkeit zu einer anderen umgeschaltet.

Zwar ist dieses Ventil zunächst nur für das Umschalten zwischen zwei Fluiden vorgesehen. Durch eine serielle Hintereinanderschaltung mehrer solcher Ventile kann jedoch auch zwischen drei und mehr Fluiden hin- und herschaltet werden.

Die beanspruchte Vorrichtung ermöglicht insbesondere, Temperaturen lokal beschränkt an einer Probe zu verändern. Dies ist bei der Schmerzforschung von Interesse.

Es zeigen
Figur 1a, b: Aufbau einer Vorrichtung zur Erzeugung von Temperaturwechseln
Figur 2a, b: Umschaltventile für Vorrichtung

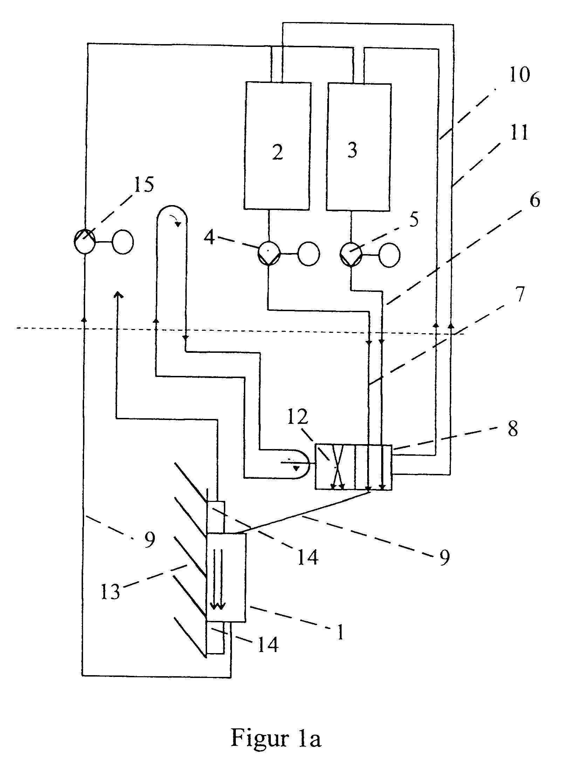

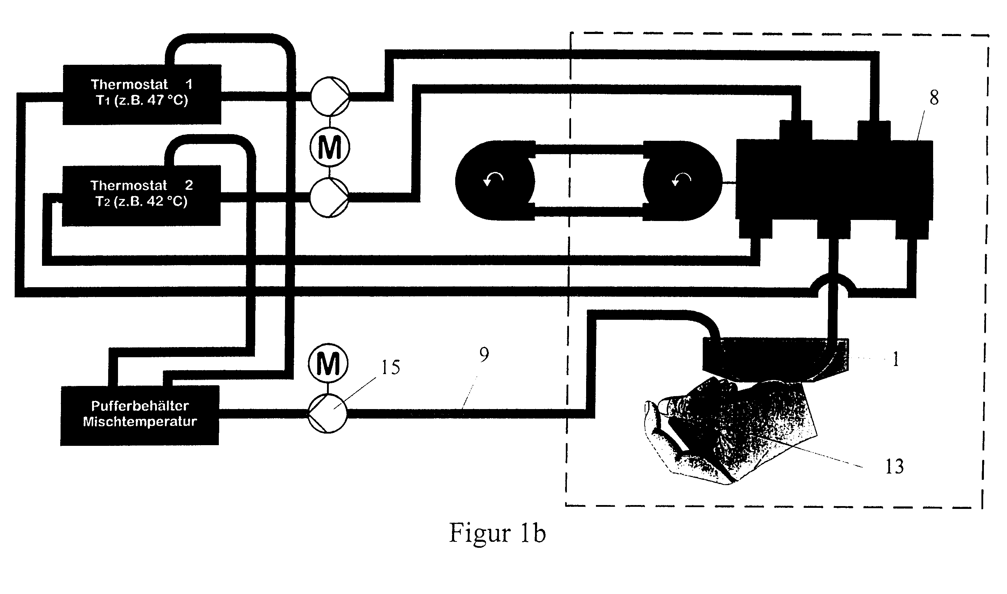

Figur 1a, b zeigt zwei Flüssigkeitsbehälter 2 und 3. Die Flüssigkeit im Behälter 2 befinde sich aufeiner Temperatur T₁ (z. B. mittels eines Thermostaten 1) und im Behälter zwei auf einer Temperatur T₂ (z. B. mittels eines Thermostaten 2). T₁ sei verschieden von T₂. Mittels der Pumpen 4 und 5 werden die Flüssigkeiten über die Schläuche 6 und 7 zur Einrichtung 8 transportiert. In der Einrichtung 8 seien die Umschaltmittel 12 zunächst so geschaltet, daß die aus dem Flüssigkeitsbehälter 2 kommende Flüssigkeit über die Schlauchverbindung 9 das Kontaktmittel 1 durchströmt. Hierdurch wird die Probe 13 auf die Temperatur T₁ gebracht. Anschließend fließt die Flüssigkeit zurück in ihr Reservoir 2. Die aus dem Behälter 3 stammende Flüssigkeit fließt über die Schlauchverbindung 10 zurück ins Reservoir 3.

Wird in der Einrichtung 8 mit den Mitteln 12 umgeschaltet, so fließt die aus dem Behälter 3 stammende Flüssigkeit durch den Kontaktkopf 1 und die Probe 13 wird auf die Temperatur T₂ gebracht. Durch geeignete Mittel fließt die Flüssigkeit dann zurück in ihren Ausgangsbehälter. Die aus dem Behälter 2 stammende Flüssigkeit fließt nach der Umschaltung über die Schlauchverbindung 11 zurück in ihren Ausgangsbehälter.

Der Kontaktkopf 1 besteht aus einem Behälter, der zur Probe 13 hin offen ist. Wird mittels der Pumpe 15 derart im Verhältnis zu Pumpe 4 bzw. 5 gepumpt, daß im Kontaktkopf 1 ein Unterdruck entsteht, so bewirkt der Unterdruck die Fixierung des Behälters an der Probe 13. Verstärkt wird dieser Effekt durch einen zusätzlich angebrachten, umlaufenden Behälter 14, der in gleicher Weise funktioniert. In diesem Umlaufbehälter befinde sich beispielsweise Luft, die abgesaugt wird.

Sämtliche Geräte, die elektrische oder magnetische Felder erzeugen, können problemlos vom Meßort ferngehalten und abgeschirmt werden. Mechanische Störungen werden durch den Einsatz vibrationsarmer Ventile vermieden.

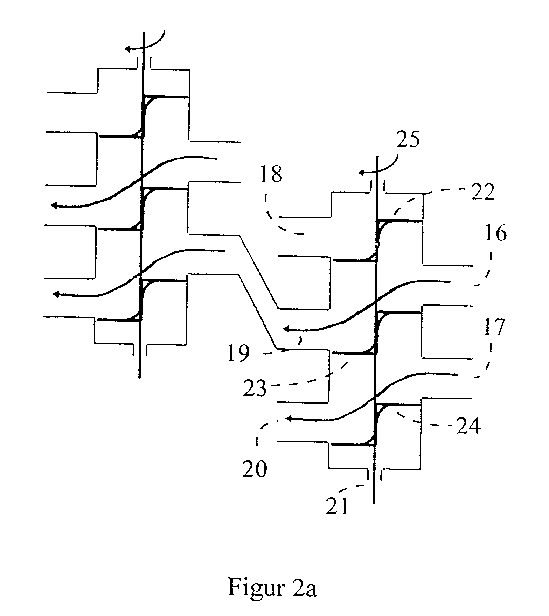

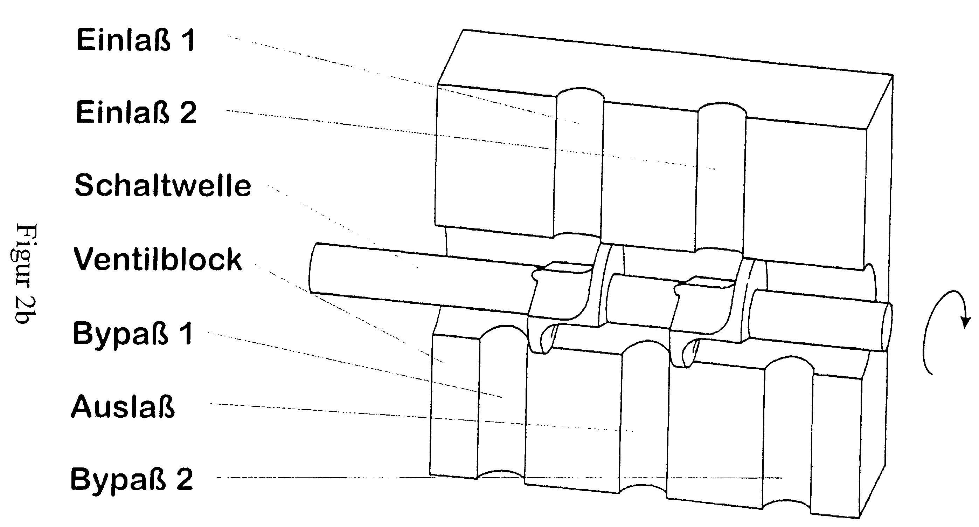

Als vibrationsarmes Umschaltmittel wird ein Ventil gemäß Figur 2a, b eingesetzt.

Figur 2a zeigt einen Schnitt durch ein Ventil zum Umschalten zwischen zwei Fluiden. Das Ventil weist Einlaß- 16,17 und Auslaßöffnungen 18, 19, 20 längs zu einer im Ventil befindlicher Welle 21 auf. An der Welle 21 sind Leitbleche 22, 23, 24 (z. B. in Form von Taumelscheiben) derart an der Welle angebracht, daß in das Ventil hineinströmende Fluide durch eine 180°-Drehung (Drehung wird durch Pfeil 25 angedeutet) der Welle 21 eine Umlenkung erfahren. Eine solche Umlenkung bewirkt, daß das zur Einlaßöffnung 16 hineinströmende Fluid nicht mehr durch die Auslaßöffnung 19, sondern durch die Auslaßöffnung 18 das Ventil verlaßt. Gleichzeitig verläßt das durch die Einlaßöffnung 17 hineinströmende Fluid das Ventil statt durch Auslaßöffnung 20 durch Auslaßöffnung 19. An der Auslaßöffnung 19 hat somit der gewünschte Wechsel stattgefunden.

Soll ein Wechsel zwischen drei Flüssigkeiten möglich sein, so wird ein zweites Ventil in dargestellter Weise nachgeschaltet.

Mittels solcher Ventile ist ein schnelles Hin- und Herschalten problemlos möglich.

Figur 2b zeigt eine dreidimensionale Darstellung des Ventils.

Das zur Zeit gebaute Gerät hat eine kompakte Ventil-Kontaktkopfeinheit von ca. 60 mm x 60 mm x 60 mm. Angetrieben wird das Ventil durch einen Wasserhydraulikmotor mit eigenem, vom Heizwasser unabhängigem, Wasserkreislauf. Die komplette Einheit ist hauptsächlich aus den Kunststoffen PMMA, POM, PA1 1, Acrylharz sowie PTFE gefertigt. Als Schlauchmaterial wurde weich PVC verwendet, wobei sich hier nahezu jeder nicht metallische Schlauch verwenden läßt.

## Patentansprüche

1. Vorrichtung zur Erzeugung von Temperaturwechseln an einer Probe (13) mittels eines Kontaktmittels (1),
wobei Mittel zur getrennten Temperierung von Gasen oder Flüssigkeiten (2, 3), zur getrennten gleichzeitigen Zuleitung (4, 5, 6, 7) der temperierten Gase oder Flüssigkeiten in eine Einrichtung (8) sowie
zur getrennten gleichzeitigen Ableitung (9, 10, 11) der Gase oder Flüssigkeiten aus der Einrichtung (8) heraus vorgesehen sind,
die Einrichtung Umschaltmittel (12) zur Umschaltung der Verbindungen zwischen Zuleitungs- und Ableitungsmitteln aufweist und
eines der Ableitungsmittel (9) zum Transport eines der temperierten Gase oder Flüssigkeiten durch das Kontaktmittel (1) vorgesehen ist.

2. Vorrichtung nach vorhergehendem Anspruch,
dadurch gekennzeichnet, daß
als Kontaktmittel ein zu einer Seite hin offener Behälter sowie Befestigungsmittel (14, 15) zur Befestigung der offenen Seite des Behälters an der Probe (13) vorgesehen sind.

3. Vorrichtung nach vorhergehendem Anspruch,
dadurch gekennzeichnet, daß
als Befestigungsmittel Mittel zum Herauspumpen (15) von Gasen oder Flüssigkeiten an den Behälter angeschlossen sind.

4. Vorrichtung nach einem der vorhergehenden Ansprüche,
gekennzeichnet durch
Ventile als Umschaltmittel (12) zum Umschalten zwischen Fluiden, mit Einlaß- (16, 17) und Auslaßöffnungen (18, 19, 20) entlang zu einer im jeweiligen Ventil befindlichen Welle (21) sowie mit derart an der Welle angebrachten Leitblechen (22, 23, 24), daß in das jeweilige Ventil hineinströmende Fluide durch Drehen (Pfeil 25) der Welle zu einer anderen Auslaßöffnung als vor der Drehung umgelenkt werden können.
